Europäisches Patentamt

(19) European Patent Office

Office européen des brevets

(11) Publication number: **0 016 887**
**B1**

# (12) EUROPEAN PATENT SPECIFICATION

(45) Date of publication of patent specification: **14.03.84**

(51) Int. Cl.³: **A 61 L 2/20, A 61 L 2/24**

(21) Application number: **79303050.3**

(22) Date of filing: **24.12.79**

(54) Gas sterilization method and apparatus.

(30) Priority: **06.02.79 US 9818**

(43) Date of publication of application:
**15.10.80 Bulletin 80/21**

(45) Publication of the grant of the patent:
**14.03.84 Bulletin 84/11**

(84) Designated Contracting States:
**AT DE FR GB SE**

(56) References cited:
**DE - A - 1 148 704**
**DE - A - 1 642 160**
**US - A - 3 954 406**
**US - A - 4 164 538**

**"Remingtons" 15th Edition (1975) pp. 1394-5**

(73) Proprietor: **American Sterilizer Company**
**2424 West 23rd Street**
**Erie, Pennsylvania 16512 (US)**

(72) Inventor: **Baran, Walter John**
**312 Maryland Avenue**
**Erie Pennsylvania 16505 (US)**

(74) Representative: **Jones, Colin et al,**
**W.P. THOMPSON & CO. Coopers Building Church**
**Street**
**Liverpool L1 3AB (GB)**

Courier Press, Leamington Spa, England.

## Gas sterilization method and apparatus

This invention is concerned with gas sterilization methods and apparatus and, more particular, with improved conditioning of goods for biocidal gas sterilization.

In conditioning goods for ethylene oxide sterilization, desired temperature and moisture conditions should be achieved throughout the goods to be sterilized. In the past, this has required extended time periods, or provision of conditioning materials and energy often substantially in excess of that needed for heating and moistening the load, or instrumentation which required operator steps or instruments which may not remain reliable under sterilizing conditions.

In the prior practice, often referred to as the static method of conditioning, as described in US—A—3068064, moisture is added and held in an otherwise sealed chamber while the goods are heated by the chamber walls. This approach can take extended periods of time, especially with soft goods loads, for conditions to exist in the load for an optimum kill rate of microorganisms; therefore, extended sterilization cycle times result.

In other prior practice, described as dynamic conditioning, as described in US—A—3598516, steam flows through the chamber while the chamber is being evacuated. Heating and moistening of the goods are expedited and total sterilization cycle times are thereby shortened; but large amounts of moisture and heat are pumped from the chamber during the conditioning phase; also, checks on conditioning operation have not been readily available, selection of conditioning values is minimal, and the opportunities for developing wet packs with certain types of loads are greater than desired.

DE—A—1642160 describes an ethylene oxide sterilizing processes and apparatus in which a steam conditioning phase is preceded by an evacuation phase. In the process of Figure 2 of this publication, there is illustrated a slight modification to the dynamic approach in which vacuum is applied simultaneously with the controlled introduction of steam during the major part of the evacuation phase in order that the steam will purge the residual air from the chamber. Figure 1 of this publication, on the other hand, shows the conventional static approach in which the evacuation phase is accelerated or enhanced by repeated evacuation and steam introduction steps. DE—A—1642160 does not disclose the application of vacuum during the steam treatment phase.

More recently, conditioning approaches have been suggested in which temperature and humidity sensors are embedded in the goods to be sterilized for the purpose of signalling conditions within the goods; such approaches can have inherent disadvantages in the accuracy and functioning of sensors and requires placement of the sensors by the operator before start of the cycle. In any of the prior art, the imposition of conditioning stem flow rate requirements can cause difficulties in achieving proper control and in maintenance and service requirements.

An object of the invention is to substantially reduce or eliminate, by practical minimizing of conditioning vapour, instrumentation and flow rate control requirements while providing effective and efficient conditioning of the goods to be sterilized.

Another object of the invention is to eliminate any requirement for humidity sensing equipment or temperature sensing equipment in the load or in the sterilizing chamber, for purposes of control of conditioning. A further object is to provide a cycle in which conditioning vapour injection is independent of rate control requirements so that prior art requirements for needle valves or other flow rate control devices for conditioning steam can be eliminated.

The present invention resides in a method of sterilizing goods with a biocidal gas, such as ethylene oxide gas, in a sealable chamber, in which the goods in the chamber are conditioned prior to the introduction of the biocidal gas by initially evacuating the chamber to a lower sub-atmospheric pressure, intermittently introducing a conditioning vapour, such as steam, into the chamber, the chamber being re-evacuated prior to each such introduction, and holding the conditioning vapour in the chamber for a predetermined time in intimate contact with the goods at a higher sub-atmospheric pressure with the evacuation and the vapour introduction both interrupted and whilst maintaining the goods substantially at a desired temperature for conditioning, characterized in that the conditioning phase at said higher sub-atmospheric pressure is interrupted several times at predetermined time intervals, each interruption comprising the steps of re-connecting the chamber to the source of vacuum, closing the connection between the source of vacuum and the chamber responsively to the chamber pressure reaching said lower-sub-atmospheric pressure, re-connecting the source of conditioning vapour to the chamber without controlling the rate of flow of conditioning vapour into the chamber, and closing the connection between the source of conditioning vapour and the chamber responsively to the chamber pressure again reaching said higher sub-atmospheric pressure.

Thus a conditioning drive power, which increases load conditioning efficiency, is provided by controllably changing chamber pressures to facilitate early attainment and control of moisture and heat requirements within the load. In contrast to the dynamic conditioning of the prior art, conditioning material and energy are conserved with most loads and the likelihood of wet packs substantially reduced or eliminated. Also, total sterilization cycle times are considerably shortened over those available with static methods of the prior art and do not exceed those available with the prior art dynamic conditioning methods, and verification of actual conditioning, while in process, is readily available.

Optimum kill-rate temperature and humidity conditions are established in the load prior to addition of the biocidal gas. The desired load temperature established during conditioning and gas charge can be readily maintained in the chamber during the sterilizing phase. The moisture requirements for sterilization established during conditioning are held within the chamber during sterilization. Conditioning prior to adding biocidal gas enables sterilization to take place in shorter time periods utilizing generally accepted ethylene oxide concentrations of about 600 to 700 mg. of ethylene oxide (ETO) per litre or sterilizing chamber volume at pressures from about 35,000 to 70,000 Pa gauge of a mixture of 12% ETO and 88% diluent gas.

With conditioning and ethylene oxide concentrations established, sterilization times are dependent on the temperature selected for the cycle. The present invention can provide for sterilization cycles at differing temperatures within the range accepted as practical, generally above about 40°C. to about 75°C. for so-called "cold" sterilization; and, additionally, the method of the invention is adaptable to a variety of sterilizing chamber sizes and chamber loads.

Following initial evacuation, the conditioning phase includes a plurality of repressurizations accomplished by injection of a conditioning vapour, typically steam from a supply at a pressure of about $2.4 \times 10^5$ to $5.5 \times 10^5$ Pa gauge (35 to 80 psig), having heat transfer and moistening properties. Initial evacuation to a subatmospheric pressure level, preferably in the range of $6.7 \times 10^3$ to $9.3 \times 10^3$ Pa (50 to 70 mm Hg), which is below that corresponding to the desired sterilization temperature, preferably by $2 \times 10^3$ to $4 \times 10^3$ Pa (15 to 30 mm Hg), is followed by repressurization to a higher subatmospheric pressure level corresponding to the pressure level for the vapour which establishes approximately the desired sterilization temperature. Such repressurizations at subatmospheric pressure levels provide a drive power for the conditioning vapour which enhances penetration of heat and moisture into the load and are preferably repeated at least twice again. Between each repressurization and the subsequent evacuation, chamber evacuating and steam injecting are interrupted for predetermined intervals, preferably of about 3 minutes each.

The goods to be sterilized are heated and moistened by the conditioning steps and, chamber walls are preferably held at desired sterilization temperature during the sterilizing phase to avoid heat losses from the vicinity of the load.

The invention includes sterilizing apparatus for carrying out the method of the invention and comprising a sterilizing chamber a first valve for connecting a source of vacuum to the chamber, a second valve for connecting a source of conditioning vapour to the chamber, and pressure sensing means operable responsively to the chamber pressure characterised in that the second valve for the conditioning vapour is an on—off valve, and the pressure sensing means comprises pressure responsive units responsive to lower and higher subatmospheric pressures, and in that a control device is provided for closing the evacuation valve and opening the conditioning vapour valve when one pressure responsive unit responds to the lower subatmospheric pressure being reached and for closing the conditioning vapour valve when the other pressure responsive unit responds to the higher sub-atmospheric pressure being reached, and furthermore in that the control device contains a timer which opens the evacuation valve after the elapse of each time interval during the conditioning phase.

The invention is further described, by way of example, with reference to the accompanying drawings; in which:—

Figure 1 is a pressure vs. time graph representation of a cycle including evacuating conditioning, sterilizing, and exhaust phases by which a preferred embodiment of the method of the invention is carried out;

Figure 2 is a schematic diagram of sterilizing apparatus embodying the invention;

Figure 3 is a larger scale view of a portion of the graph of Figure 1 showing a specific embodiment of an initial evacuation phase; and

Figure 4 is a schematic circuit diagram of a preferred control device in the apparatus of the invention.

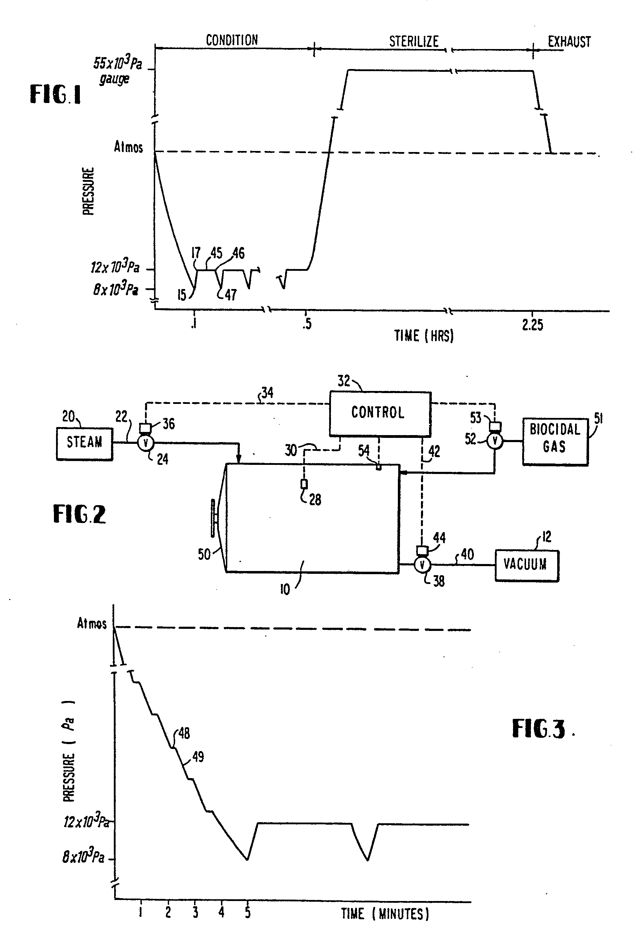

For purposes of a detailed description, an embodiment of the invention carried out at about 54°C. (130°F.), a temperature widely used for sterilizing various loads including hard goods, fabrics, and plastics, is presented. A shown in Figure 2, a pressure vessel 10 is connected to vacuum source 12 for evacuation to desired subatmospheric pressures. As indicated at point 15 in Figure 1, the chamber is initially evacuated to a subatmospheric pressure level of about $8 \times 10^3$ Pa (60 mm Hg).

A conditioning vapour capable of transferring latent heat through condensation, such as steam, is fed from source 20 through conduit 22 to the chamber under the control of on/off valve 24. Injection of this vapour raises the pressure to a second selected higher subatmospheric pressure of $12 \times 10^3$ Pa (90 mm Hg.) as indicated by point 17 in Figure 1; this pressure corresponds approximately to the temperature desired for sterilization as determined by the interrelationship of pressure and temperature available from steam or other vapour tables.

A significant feature of the method is provision for the conditioning vapour used to be injected without requiring injection rate control of the vapour additions; for example, steam can be used at available pressures, typically from about $3.5 \times 10^5$ Pa to $5.5 \times 10^5$ Pa gauge, without requiring needle valve or other flow rate controls, thus eliminating possibly troublesome devices.

In the 54°C. cycle, chamber pressure is reduced during the initial evacuation to the lower of two selected subatmospheric pressures, about $8 \times 10^3$ Pa, then raised to the subsequent higher subatmospheric pressure of about $12 \times 10^3$ Pa. The second higher subatmospheric pressure selected should provide a temperature around 52°C. (125°F.), thus allowing for the heat of pressurization produced by adding biocidal gas above atmospheric pressure. after conditioning and compensating partially for the higher temperature which can occur within dry fabric packs when moistening and heating by condensation of the conditioning vapour.

The evacuation and repressurization levels for conditioning can be preset on pressure switch means to provide pressure responsive on/off control of vacuum and steam injection. Sensing of temperature or relative humidity are not required for control of conditioning process steps.

Chamber pressure determinations can be made with pressure sensing means 28 which sends a pressure signal through electrical connector 30 to control 32 which, in turn, sends a control signal over electrical connector 34 to solenoid 36 for on/off control of valve 24 in steam line 22.

Control 32 also actuates on/off valve 38 in vacuum line 40 through signal connector 42 interconnecting control 32 to valve operating solenoid 44. Vacuum source 12 can be a vacuum pump of the type and size standardly accepted as economically and operationally practical for the particular size chamber.

Evacuation is interrupted at $8 \times 10^3$ Pa (point 15 of Figure 1) and steam is injected. As the $12 \times 10^3$ Pa pressure is reached (at point 17 in Figure 1), steam injection is interrupted. With both steam injection and evacuation interrupted, the chamber is held in this sealed condition for a predetermined interval; for example, three minutes, as indicated by line 45 of Figure 1.

At the end of this predetermined time (point 46) during which the chamber is sealed, control 32 opens vacuum valve 38 to evacuate the chamber to approximately the initial subatmospheric pressure, about $8 \times 10^3$ Pa as indicated by point 47 in Figure 1. To facilitate handling mixed loads, a pressure differential of $4 \times 10^3$ Pa is selected but a smaller pressure change can be satisfactory under certain load conditions.

The sequence of evacuating to about $8 \times 10^3$ Pa, repressurizing to about $12 \times 10^3$ Pa, and then holding the chamber without evacuating or injecting steam for a predetermined interval is repeated. The number of these repeated sequences is preselected to provide desired conditioning prior to the sterilization phase; for example, in a 54°C. cycle, with a vacuum pump rated at about 3.3 $ls^{-1}$ used on a sterilizer whose chamber measures from about 0.4 m³ to about 0.8 m³, repressurization is set occur five times; with a vacuum pump rated at about 0.33 $ls^{-1}$ used on a sterilizer chamber between about 0.2 m³ to about 0.3 m³, repressurization is set to occur three times to accomplish desired conditioning of all types of loads.

All packs in multiple pack loads are conditioned for temperature and humidity prior to admission of the biocidal gas. Mixed full loads included cardboard box packs, obstetrics packs, Canadian Standards Association packs, a USA Federal specification pack, an Emergency Caesarian pack, paper/plastic peel pouches, and a plastic bedpan subpack. Biocidal gas (12% ETO, 88% diluent gas) held at a pressure of $5.5 \times 10^4$ Pa gauge to provide approximately 650 mg. per litre of ETO concentration provides 100% kill of Bacillus subtilii (globigii) with an exposure time of 105 minutes which includes an adequate safety factor assuring sterilization for hospital practice. With such mixed full loads, and with single pack loads, all packs were heated to approximately desired sterilization temperature and humidified during the conditioning phase to approximately 60% to 100% RH.

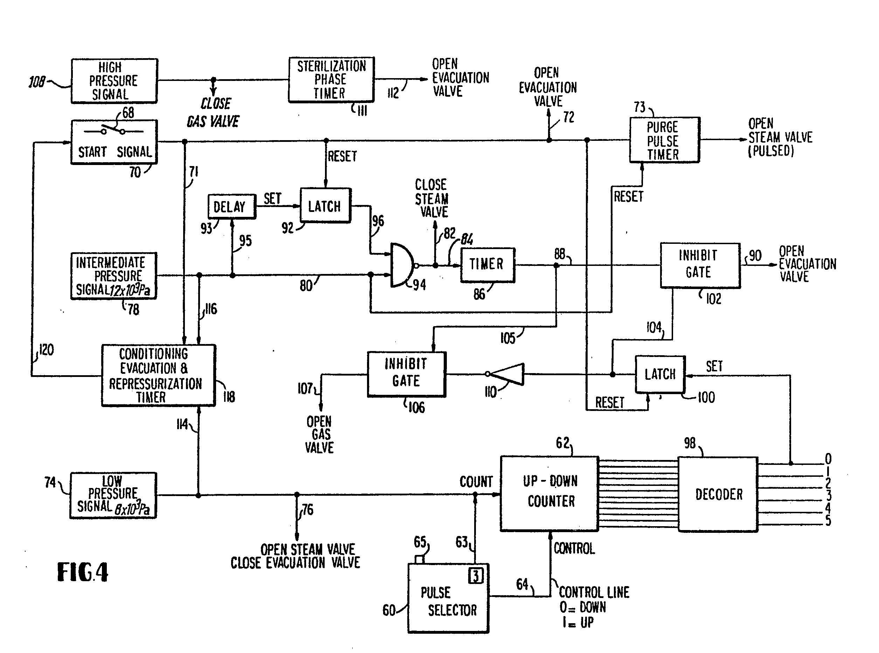

In the embodiment of Figure 3, the steam valve 24 is opened intermittently during initial evacuation. In a typical pulsing arrangement, steam is added in five (5) second pulses (indicated by reference numeral 48 in Figure 3) at thirty (30) second intervals (indicated by reference numeral 49). This steam purge arrangement helps provide for more rapid load conditioning to temperature. Evacuation continues during the short steam injection purge pulses; in the later vapour injection phases, evacuating is terminated to reduce heat and energy losses.

The short pulse injection method enables use of a vapour injection purge which contributes to the versatility of the cycle. Use of vapour injection during initial evacuation to facilitate heat-up is especially suited to cycles in the upper portion of the commerical practical ethylene oxide sterilization temperature range, i.e. about 52°C. and higher. Selecting a short injection pulse followed by a substantially longer interval without steam injection enables use of on/off steam injection free of rate control over a wide range of steam pressures. Load heating efficiency is increased while avoiding overheating of the load. The ratio of injection pulse time to interval time between pulses can be about 1:5; in the 54°C. cycle with steam injection pulses taking place during the initial evacuation so far as $12\times10^3$ Pa., a ratio of 1:6 provides satisfactory results.

With cycles at the lower end of the accepted practical emperature range for ethylene sterilization, e.g., above 38°C. to less than 52°C., steam purge pulses would generally not be utilized because it would not be practical to provide the evacuating capacity needed to avoid overheating items which might be sterilized at such low temperatures. As the desired cyclic temperature increases, the steam pulsing purge can be limited to later portions of the initial evacuation when injected steam temperature more closely approaches desired sterilization temperature. The vapour injection purge pulses generally terminate during the initial evacuation as the $12\times10^3$ Pa. pressure level is reached.

The on/off valve 24 is the only valve required in the conditioning steam line 20 for flow purposes (a check valve can be used for other purposes). Major advantages of the vapour injection in the method and apparatus of the invention include elimination of needle valves on conditioning steam lines; this eliminates needle valve clogging problems and needle valve adjustment problems in usage. Not only is this potential problem area eliminated but on/off steam flow facilitates rapid verification that conditioning vapour is being injected.

With the pressure responsive control in the method of the invention, verification of conditioning can be readily obtained since conditioning vapour injection is required to establish pressure relationships in the chamber which are utilized in determining whether the cycle is to proceed. Subatmospheric repressurization times longer than a fraction of a minute, which are readily discernible, provide a check that conditioning is proceeding. This concept of conditioning verification was not available in prior practice. In the preferred embodiment of the present invention, parameters on chamber pressure vs. lapsed time can be used to indicate when conditioning is not proceeding and to abort the cycle.

Component requirements are also reduced in the control deivce of the apparatus. Timer sequencing means are utilized; however, pressure levels are the only chamber sensed determinations required for operation of conditioning steps. While chamber wall temperature can be thermostatically controlled, as in prior practice, chamber temperature sensors or relative humidity sensors are not required for the conditioning steps to proceed reliably. Also, the same steam line and variable steam pressures can be used for various temperature cycles; the present method can be effective at substantially lower steam pressures than generally available; however, cycle efficiency is better maintained at steam pressures of $2.4\times10^5$ Pa and higher.

Basically, control 32 includes elements to receive incoming pressure signals and, through timer and sequencing means, coordinates on/off valve operations and times. After chamber 10 is loaded with goods to be sterilized, closure 50 seals the chamber for operation at pressures other than atmospheric. After completion of the conditioning phase, as described, the chamber is charged with a biocidal gas from source 51 under control of valve 52 which is actuated by solenoid 53.

Pressure sensing means 28 can be divided for practical purposes, i.e. to provide accuracy with economy, into a pressure switch comprising signal units 74, 78 (Figure 4) operable at the subatmospheric levels of $8\times10^3$ Pa and $12\times10^3$ Pa, and a pressure switch operable at superatmospheric pressures of about $1.7\times10^5$ Pa as used for biocidal gas. To minimize heat losses during the sterilization phase, the sterilizer chamber walls can be maintained at about 54°C. through thermostat 54 by electrical strip heaters or by a steam jacket.

Solenoid-operated valves, pressure sensors, timer and sequencing means, and steam, gas, and vacuum sources described above are commercially available and need no further description. The present combination of these individual elements effects conditioning control while eliminating the need for load contact sensors, chamber relative humidity gauges, and needle valve type flow control of steam injection.

The various chamber evacuation and injection means are controlled relying ultimately only on sensing chamber pressure and time measurements which provide for continuing reliability. Initial evacuation is pressure responsive and purge vapour injection pulses are timer controlled. Pulse evacuation and repressurization phases are pressure responsive and intermediate intervals are timer controlled. Charging biocidal gas and sterilizing gas pressure are pressure responsive. Measured pressures and time intervals can be combined by electromechanical or other electrical control elements.

In the electronic control device of Figure 4, the number of repressurization sequences can be preset and fixed before the process is begun for a particular sterilizer or selected by operating or service personnel. The desired number of repressurization pulse sequences is selected at pulse selector 60 which loads up-down counter 62 accordingly. A pulse is generated on both output lines 63, 64 by the pulse selector circuit each time button 65 is depressed. The counter can be operated in two modes and is arranged so that, when its control input is a zero (low or no signal), the counter counts down and, when its input is a "one" (high signal), the counter counts up. Since control line 64 will be a "one" (high signal) each time a pulse is fed from pulse selector 60 to the count input of up-down counter 62, the counter will up count to a number corresponding to the number of pressurization sequences selected at button 65.

With the up-down counter 62 preloaded, the cycle is started by closing switch 68 of start signal generator 70. This generates an electrical signal on line 72 which opens the evacuation valve 38 of Figure 2 through solenoid valve 44.

Initial evacuation is continuous to a preselected subatmospheric level and can include a series of short time interval steam pulses as shown in the graph of Figure 3. When the vapour injection purge is used, steam pulse timer 73 provides pulses operation of solenoid 36 to open and close steam valve 24 of Figure 2 during initial evacuation from atmospheric pressure down to $12 \times 10^3$ Pa as shown in Figure 3 and previously described. Pulse timer 73 is reset from intermediate signal unit 78 over line 80 to be operative only during the initial evacuation.

In the 54°C. cycle described, evacuation continues after the initial purge evacuation until the pressure in the chamber reaches $8 \times 10^3$ Pa, whereupon low pressure signal unit 74 generates an output signal. This output signal on line 76 is effective to close the evacuation valve 38 and open the steam valve 24. Steam injection causes the pressure in the chamber to rise to $12 \times 10^3$ Pa, (point 17 in the graph of Figure 1). When the chamber pressure reaches $12 \times 10^3$ Pa, intermediate pressure signal unit 78 generates an output signal on line 80 which is effective, via line 82, to close the steam valve 24 and, via line 84, to activate timer 86.

After the three-minute delay during which both steam injection and evacuation are interrupted, a signal from timer 86 on line 88 is effective to open the evacuation valve again by a signal on line 90. When the pressure is reduced to $8 \times 10^3$ Pa again, the evacuation valve 38 is closed and the steam valve 24 is opened; and sequencing as described above continues until the conditioning phase is ended by completion of the preselected number of sequences.

Additional means are provided for suppressing the signal from intermediate pressure signal unit 78 when the $12 \times 10^3$ Pa pressure level is first reached during the initial evacuation. This may be effected by providing latch 92, delay means 93, and AND gate 94. The first time that the pressure falls to the intermediate pressure level ($12 \times 10^3$ Pa) and intermediate pressure signal unit 78 generates a signal, such signal is fed on line 80 to a first input terminal of AND gate 94 and, simultaneously, is fed on line 95 to delay network 93 which is set to delay the signal by a fraction of a sequence. After the signal is delayed, it is fed to the set input of latch 92 thereby setting the latch. The output of the latch 92 is fed on line 96 to the second input terminal of AND gate 94. However, due to the delay, it does not arrive at the AND gate until the first output signal of intermediate pressure signal units 78 has disappeared from the first input to the AND gate. Therefore, the first output signal does not pass through the AND gate and is not effective to close the steam valve or to set the timer 86. However, since the latch 92 remains set for all subsequent sequences in the conditioning phase, all output signals of intermediate pressure signal unit 78 after the first signal pass through AND gate 94 and are effective to close the steam valve and to set the timer 86 to carry out the prescribed pulsing sequence.

After the last squence in the conditioning phase, biocidal gas is added to raise chamber pressure to a selected super-atmospheric pressure level such as $5.5 \times 10^4$ Pa gauge. The last conditioning sequence is detected by up-down counter 62 and decoder 98. When the counter counts down to zero, the decoder output will become high. This signal is fed to the set input terminal of latch 100, the output of which is fed to INHIBIT gate 102 on line 104. INHIBIT gate 102 is effective to pass the output signal of timer 86 at all times except when the latch 100 input to the INHIBIT gate is high. Thus, after the pressure reaches $12 \times 10^3$ Pa in the last sequence, the output of the timer 86 is prevented by INHIBIT gate 102 from being fed through to the circuit point for opening the evacuation valve. Instead, the output of the timer 86 is effective to open the gas valve 52 (Figure 2). As shown in Figure 4, this output is fed via line 105 through INHIBIT gate 106 to line 107 for opening the gas valve 52. This signal is inhibited by the output of latch 100 and inverter 110 at all times except after the last sequence when latch 100 becomes set. Both of the latches 92 and 100 are reset by the start signal generator 70 at the beginning of a conditioning phase; the start signal generator 70 can also reset the up-down counter 62 to "start" condition.

The entry of biocidal gas is continued until the chamber pressure reaches about 55000 Pa gauge, whereupon high pressure signal unit 108 generates a signal which is effective to close the gas valve 52 through solenoid 53 (Figure 2) and, after a time delay equal to the sterilization phase time introduced by timer 111, is also effective to open the evacuation valve 38 by a signal on line 112; this completes the sterilization phase as depicted in Figure 1.

Means are provided to abort the cycle when transition times between the two selected subatmospheric levels exceed reasonable preset parameters. Considering a hospital sterilizer having a chamber volume of about 0.7 m³, mixed full pack, 54°C. cycle, initial vacuum to $12 \times 10^3$ Pa (with five second steam pulses at thirty second intervals) takes approximately 4 to 4.5 minutes, evacuation from $12 \times 10^3$ to $8 \times 10^3$ Pa about one minute, repressurization to $12 \times 10^3$ Pa about 30 to 45 seconds, chamber hold three minutes, evacuate to $8 \times 10^3$ Pa about 1 minute, repressurization to $12 \times 10^3$ Pa about 30 to 45 seconds, etc. through five repressurizations. Gas charge to $5.5 \times 10^4$ Pa gauge can take from about 7.5 to 15 minutes.

Sterilization time, with safety factor, is arbitrarily set at 105 minutes. Exhausting and air breaks, after sterilization, can take from 10 to 15 minutes. The total cycle time for 54°C. sterilization temperature is about 165 minutes.

In order to verify that initial evacuation and conditioning are proceeding, any of the allowable preset parameters can be checked through evacuation and repressurization timer 118. Transition times, e.g. any of the repressurization times between $8 \times 10^3$ and $12 \times 10^3$ Pa, such as between points 15 and 17 of Figure 1, during the sequencing, can be evaluated. The repressurization phase, with on/off control of steam should be less than one minute in hospital sterilizers. The time between the low pressure signal from the low pressure signal unit 74 on line 114 and the intermediate pressure signal for the intermediate pressure signal unit 78 delivered on line 116 are compared in timer 118. Excessive repressurization times cause a signal to be generated on line 120 which interrupts the cycle through start signal generator 70.

Criteria which can be met by the method of the invention include sterilization efficiency with various types of loads enabling mixing of load materials (hard goods, fabrics, rubber and plastic material, and instruments), avoidance of wet packs or damaged loads, and compliance with governmental test specifications (USA Fed Spec GG—S—1344A—11/26/75). Content data for the Canadian Standards and USA Federal Specification Packs are published; in addition, the mixed full loads tested included two cardboard filled boxes weighing approximately 6.8 Kg apiece, two boxes of OB pads weighing approximately 4.5 Kg each, two wrapped plastic bedpans, an emergency Caesarian pack, and paper/plastic pouches containing surgical or anesthesia equipment. Conditioning to desired moisture and temperatures was verified in packs, previously considered difficult to heat, with five sequences for such mixed full load.

Biological spore strips, tested to assure a minimum average population per strip of $1 \times 10^6$ spores of Bacillus subtilis (globigii), were used with 100% kill of microorganisms consistently obtained. At 54°C., a 105 minute gas exposure period provides adequate safety factor to meet hospital specifications. With baffling to prevent direct impingement of incoming steam onto the load, wet packs are avoided and load conditioning is consistently completed before gas charge. The sterilization phase times can be decreased at higher temperatures and increased at lower temperatures in accordance with established skills in gas sterilization.

## Claims

1. A method of sterilizing goods with a biocidal gas, such as ethylene oxide gas, in a sealable chamber, in which the goods in the chamber are conditioned prior to the introduction of the biocidal gas by initially evacuating the chamber to a lower sub-atmospheric pressure, intermittently introducing a conditioning vapour, such as steam, into the chamber, the chamber being re-evacuated prior to each such introduction, and holding the conditioning vapour in the chamber for a predetermined time in intimate contact with the goods at a higher sub-atmospheric pressure with the evacuation and the vapour introduction both interrupted and whilst maintaining the goods substantially at a desired temperature for conditioning, characterized in that the conditioning phase at said higher sub-atmospheric pressure is interrupted several times at predetermined time intervals, each interruption comprising the steps of re-connecting the chamber to the source of vacuum, closing the connection between the source of vacuum and the chamber responsively to the chamber pressure reaching said lower sub-atmospheric pressure, re-connecting the source of conditioning vapour to the chamber without controlling the rate of flow of conditioning vapour into the chamber, and closing the connection between the source of conditioning vapour and the chamber responsively to the chamber pressure again reaching said higher subatmospheric pressure.

2. Method according to claim 1, in which the conditioning vapour comprises steam, and in which the lower sub-atmospheric pressure to which the chamber is evacuated is in the range of about $6.7 \times 10^3$ to $9.3 \times 10^3$ Pa.

3. Method according to claim 2, in which the pressure increase achieved by introducing the conditioning vapour to raise chamber pressure to the higher subatmospheric pressure is in the range of about $2 \times 10^3$ to $4 \times 10^3$ Pa.

4. Method according to claim 2 or 3, in which the steam is drawn from a supply at a pressure of about $2.4 \times 10^5$ to $5.5 \times 10^5$ Pa gauge.

5. Method according to claim 2, 3 or 4, in which the predetermined time intervals during which the chamber is held in sealed condition with evacuation and steam introduction interrupted are each about three minutes.

6. Method according to any preceding claim, in which a time limit for repressurizing the chamber between the lower and higher sub-atmospheric pressures is established and the conditioning and sterilizing cycle is aborted if the repressurizing time during a sequence exceeds such preselected time limit.

7. Method according to any preceding claim, in which conditioning vapour is admitted into the chamber during at least one portion of said initial evacuation to remove air.

8. Method according to claim 7, in which the conditioning vapour is admitted during such initial evacuation by intermittently injecting the conditioning vapour to provide a plurality of short vapour injection pulses as the chamber is being evacuated.

9. Method according to claim 8, in which such intermittent injections of condensable vapour are free of flow rate control of the conditioning vapour, and in which the ratio of the time duration of the vapour injection pulses to the time interval between such injection pulses during such chamber purge phase is such as to avoid overheating the goods substantially above desired sterilization temperature.

10. Method according to any preceding claim, in which the temperature of the goods to be sterilized is maintained by maintaining a chamber wall portion at approximately the desired sterilization temperature while biocidal gas is in the chamber.

11. Sterilizing apparatus for carrying out the method claimed in any preceding claim, comprising a sterilizing chamber (10), a first valve (38) for connecting a source of vacuum (12) to the chamber (10), a second valve (24) for connecting a source (20) of conditioning vapour to the chamber (10), and pressure sensing means (28) operable responsively to the chamber pressure characterised in that the second valve (24) for the conditioning vapour is an on—off valve, and the pressure sensing means (28) comprises pressure responsive units (74, 78) responsive to lower and higher sub-atmospheric pressures, and in that a control device (32) is provided for closing the evacuation valve (38) and opening the conditioning vapour valve (24) when one pressure responsive unit (74) responds to the lower sub-atmospheric pressure being reached and for closing the conditioning vapour valve (24) when the other pressure responsive unit (78) responds to the higher sub-atmospheric pressure being reached, and furthermore in that the control device (32) contains a timer (86) which opens the evacuation valve (38) after the elapse of each time interval during the conditioning phase.

12. Sterilizing apparatus according to claim 11, in which the control device (32) contains a further timer (118) for interrupting operation of the apparatus if repressurization during conditioning steps exceeds a preselected maximum time interval.

**Patentansprüche**

1. Verfahren zum Sterilisieren von Waren mit einem biodiden Gas, wie Äthylenoxidgas, in einer abdichtbaren Kammer, bei welchem die Waren in der Kammer vor dem Einführen des biozidien Gases konditioniert werden durch anfängliches Evakuieren der Kammer auf einen niedrigeren unteratmosphärischen Druck, unterbrochenes Einführen eines konditionierenden Dampfes, wie Wasserdampf, in die Kammer, wobei die Kammer vor jedem solchen Einführen widerevakuiert wird, und durch inniges Kontakthalten des Konditionierungsdampfes mit den Waren in der Kammer während einer vorbestimmten Zeit bei einem höheren unteratmosphärischen Druck mit Unterbrechung sowohl der Evakuierung als auch der Dampfeinführung, und gleichzeitiges Halten der Waren auf einer im wesentlichen für die Konditionierung gewünschten Temperatur, dadurch gekennzeichnet, daß die Konditionierungsphase bei besagtem höheren unteratmosphärischen Druck mehrmals in vorbestimmten Zeitintervallen unterbrochen wird, jede Unterbrechung umfassend die Schritte des Wiederverbindens der Kammer mit der Vakuumquelle, des Abschließens der Verbindung zwischen der Vakuumquelle und der Kammer als Reaktion auf den den besagten niedrigeren unteratmosphärischen Druck erreichenden Kammerdruck, des Widerverbindens der Quelle des Konditionierungsdampfes mit der Kammer ohne Regelung der Einströmrate des Konditionierungsdampfes in die Kammer, und des Abschließens der Verbindung zwischen der Quelle des Konditionierungsdampfes und der Kammer in Reaktion auf den wieder den besagten höheren unteratmosphärischen Druck erreichenden Kammerdruck.

2. Verfahren nach Anspruch 1, wobei der Konditionierungsdampf Wasserdampf ist, und wobei der niedrigere unteratmosphärische Druck, bis zu welchem die Kammer evakuiert wird, im Bereich von ungefähr $6.7 \times 10^3$ bis $9.3 \times 10^3$ Pa liegt.

3. Verfahren nach Anspruch 2, wobei der durch Einführen des Konditionierungsdampfes erreichte Druckanstieg zum Erhöhen des Kammerdruckes auf den höheren unteratmosphärischen Druck im Bereich von ungefähr $2 \times 10^3$ bis $4 \times 10^3$ Pa liegt.

4. Verfahren nach Anspruch 2 oder 3, wobei der Wasserdampf von einer Versorgungsquelle mit einem Manometerdruck von ungefähr $2.4 \times 10^5$ bis $5.5 \times 10^5$ Pa bezogen wird.

5. Verfahren nach den Ansprüchen 2, 3 oder 4, wobei die vorbestimmten Zeitintervalle, während denen die Kammer bei unterbrochener Evakuierung und Wasserdampfeinführung in abgedichtetem Zustand gehalten wird, ungefähr je drei Minuten betragen.

6. Verfahren nach irgendeinem vorhergehenden Anspruch, wobei eine Zeitbegrenzung für das Widerunterdrucksetzen der Kammer zwischen den niedrigeren und den höheren unteratmosphärischen Drücken festgesetzt wird und der Konditionierungs- und Sterilisierungszyklus abgebrochen wird, wenn die Zeit für die Wiederunterdrucksetzung während einer Sequenz eine solche vorgewählte Zeitbegrenzung überschreitet.

7. Verfahren nach irgendeinem vorhergehenden Anspruch, wobei der Konditionierungsdampf in die Kammer während mindestens eines Teiles der besagten anfängliche Evakuierung eingelassen wird, um Luft zu entfernen.

8. Verfahren nach Anspruch 7, wobei der Konditionierungsdampf während einer solchen anfänglichen Evakuierung durch intermittierendes Einblasen des Konditionierungsdampfes eingelassend wird, um eine Mehrzahl von kurzen Dampfinjektionsimpulsen vorzusehen, wenn die Kammer evakuiert wird.

9. Verfahren nach Anspruch 8, wobei solche Injektionen kondensierbaren Dampfes unabhängig von der Regelung der Zuflußrate des Konditionierungsdampfes sind, und wobei das Verhältnis der Zeitdauer der Dampfinjektionsimpulse zu dem Zeitintervall zwischen solchen Injektionsimpulsen während einer solchen Kammerreinigungsphase derart ist, daß eine wesentliches Überhitzen der Waren über die gewünschte Sterilisationstemperatur vermieden wird.

10. Verfahren nach irgendinem vorhergehenden Anspruch, wobei die Temperatur der zu sterilisierenden Waren aufrechterhalten wird durch Halten eines Kammerwandteiles auf einer der gewünschten Sterilisierungstemperatur angenäherten Temperatur, während biozides Gas in der Kammer ist.

11. Steriliserungsapparat zur Durchführung des Verfahrens nach irgendeinem vorhergehenden Anspruch, umfassend eine Sterilisierungskammer (10), ein erstes Ventil (38) für den Anschluß einer Vakuumquelle (12) an die Kammer (10), ein zweites Ventil (24) für den Anschluß einer Quelle von Konditionierungsdampf an die Kammer (10), und auf Druck ansprechende Mittel (28), die in Reaktion auf den Kammerdruck betätigbar sind, dadurch gekennzeichnet, daß das zweite Ventil (24) für den Konditionierungsdampf ein Auf-Zu-Ventil ist und die auf Druck ansprechenden Mittel (28) auf Druck reagierende Elnheiten (74, 78) umfassen, die auf die niedrigeren und höheren unteratmosphärischen Drücke ansprechen, und daß eine Steuereinrichtung (32) vorgesehen ist für das Schließen des Evakuierungsventils (38) und das Öffnen des Ventils (24) für den Konditionierungsdampf, wenn eine druckabhängige Einheit (74) auf den erreichten niedrigeren unteratmosphärischen Druck anspricht, und für das Schließen des Ventils (24) für den Konditionierungsdampf, wenn die andere druckabhängige Einheit (78) auf den erreichten höheren unteratmosphärischen Druck anspricht, und daß weiterhin die Steuereinrichtung (32) einen Zeitmesser (86) enthält, welcher das Evakuierungsventil (38) nach Verstreichen eines jeden Zeitintervalls während der Konditionierungsphase öffnet.

12. Sterilisierungsapparat nach Anspruch 11, in welchem die Steuereinrichtung (32) einen weiteren Zeitmesser (118) für das Unterbrechen des Betriebs des Apparates aufweist, wenn die Wiederunterdrucksetzung während der Konditionierungsschritte ein vorgewähltes maximales Zeiteinterval überschreitet.

**Revendications**

1. Un procédé pour la stérilisation d'articles avec un gaz biocide, tel que l'oxyde d'éthylène gazeux, dans une chambre pouvant être fermée de façon étanche, dans lequel les articles dans la chambre sont conditionnés avant l'introduction du gaz biocide par évacuation initiale de la chambre à une pression subatmosphérique inférieure, introduction intermittente d'une vapeur de conditionnement, telle que la vapeur d'eau dans la chambre, la chambre étant réévacuée avant chacune de ces introductions, et maintien de la vapeur de conditionnement dans la chambre pendant une période prédéterminée en contact intime avec les articles à une pression subatmosphérique supérieure, tandis que l'évacuation et l'introduction de la vapeur sont toutes deux interrompues et en maintenant les articles pratiquement à une température désirée pendant le conditionnement, caractérisé par le fait que la phase de conditionnement à ladite pression subatmosphérique supérieure est interrompue plusieurs fois à des intervalles de temps prédéterminés, chaque interruption comprenant les stades de reconnection de la chambre à la source de vide, fermeture de la connection entre la source de vide et la chambre en réponse au fait que la pression de la chambre atteint ladite pression subatmosphérique inférieure, reconnection de la source de vapeur de conditionnement à la chambre sans commander le débit d'écoulement de la vapeur de conditionnement dans la chambre et fermeture de la connection entre la source de vapeur de conditionnement et la chambre en réponse au fait que la pression de la chambre atteint à nouveau ladite pression subatmosphérique supérieure.

2. Procédé selon la revendication 1, dans lequel la vapeur de conditionnement comprend de la vapeur d'eau et dans lequel la pression subatmosphérique inférieure à laquelle la chambre est évacuée est dans la gamme d'environ $6,7 \times 10^3$ à $9,3 \times 10^3$ Pa.

3. Procédé selon la revendication 2, dans lequel l'élévation de pression obtenue par l'introduction de la vapeur de conditionnement pour élever la pression de la chambre à la pression subatmosphérique supérieure est dans la gamme d'environ $2 \times 10^3$ à $4 \times 10^3$ Pa.

4. Procédé selon la revendication 2 ou 3, dans lequel la vapeur d'eau est fournie par une alimentation à une pression d'environ $2,4 \times 10^5$ à $5,5 \times 10^5$ Pa manométriques.

5. Procédé selon la revendication 2, 3 ou 4, dans lequel les intervalles de temps prédéterminés pendant lesquels la chambre est maintenue fermée, avec interruption de l'évacuation et de l'introduction de la vapeur d'eau, sont chacun d'environ 3 minutes.

6. Procédé selon l'une des revendications 1 à 5, dans lequel une limite de temps pour la remise en pression de la chambre entre les pressions subatmosphériques inférieure et supérieure est établie et le cycle de conditionnement et de stèrilisation est annulé si le temps de remise sous pression pendant une séquence dépasse ladite limite de temps préchoisie.

7. Procédé selon l'une des revendications 1 à 6, dans lequel la vapeur de conditionnement est admise dans la chambre pendant au moins une portion de ladite évacuation initiale pour éliminer l'air.

8. Procédé selon la revendication 7, dans lequel la vapeur de conditionnement est admise pendant cette évacuation initiale par injection intermittente de la vapeur de conditionnement pour créer plusieurs impulsions brèves d'injection de vapeur lorsque la chambre est en cours d'évacuation.

9. Procédé selon la revendication 8, dans lequel ces injections intermittents de vapeur condensable ne sont pas soumises à une commande du débit de la vapeur de conditionnement et dans lequel le rapport de la durée des impulsions d'injection de vapeur à l'intervalle de temps entre ces impulsions d'injection pendant cette phase de purge de la chambre est tel qu'on évite une surchauffe des articles nettement au-dessus de la température de stérilisation désirée.

10. Procédé selon l'une des revendications 1 à 9, dans lequel la température des articles à stériliser est maintenue par maintien d'une portion de la paroi de la chambre approximativement à la température de stérilisation désirée, tandis que le gaz biocide est dans la chambre.

11. Appareil de stérilisation pour la mise en pratique du procédé revendiqué dans l'une des revendications 1 à 10, comprenant une chambre de stérilisation (10), un premier robinet (38) pour raccorder une source de vide (12) à la chambre (10), un second robinet (25) pour raccorder une source (20) de vapeur de conditionnement à la chambre (10) et un capteur de pression (28) pouvant fonctionner en réponse à la pression de la chambre, caractérisé par le fait que le second robinet (24) pour le conditionnement de la vapeur est un robinet marche/arrêt et le capteur de pression (28) comporte des unités sensibles à la pression (74, 78) répondant aux pressions subatmosphériques inférieure et supérieure, et en ce qu'un dispositif de commande (32) est prévu pour fermer le robinet d'évacuation (38) et ouvrir le robinet de vapeur de conditionnement (24) lorsqu'une unité sensible à la pression (74) répond au fait que la pression subatmosphérique inférieure est atteinte et pour fermer le robinet de vapeur de conditionnement (24) lorsque l'autre unité sensible à la pression (78) répond au fait que la pression subatmosphérique supérieure est atteinte, et de plus en ce que le dispositif de commande (32) contient une minuterie (86) qui ouvre le robinet d'évacuation (38) après l'écoulement de chaque intervalle de temps pendant la phase de conditionnement.

12. Appareil de stérilisation selon la revendication 11, dans lequel le dispositif de commande (32) contient une autre minuterie (118) pour interrompre le fonctionnement de l'appareil si la remise sous pression pendant les stades de conditionnement dépasse un intervalle de temps maximal préchoisi.

FIG.1

FIG.2

FIG.3

FIG.4